# EUROPEAN PATENT SPECIFICATION

(11) **EP 4 031 115 B1**
(45) Date of publication and mention of the grant of the patent: **27.05.2026**
(21) Application number: 20864768.5
(22) Date of filing: 17.09.2020
(51) Int. Cl.: A61L 27/26, A61L 27/54, A61L 27/56, A61K 9/70, A61K 47/36

(54) **METHOD OF PRODUCING CRYOGELS WITH NATURAL BIOMATERIALS AND USE OF SUCH CRYOGELS AS SOFT TISSUE SCAFFOLDS OR DRUG DELIVERY SYSTEMS**
VERFAHREN ZUM PRODUZIEREN VON KRYOGELEN MIT NATÜRLICHEN BIOMATERIALIEN UND VERWENDUNG SOLCHER KRYOGELE ALS WEICHGEWEBEGERÜSTE ODER ARZNEISTOFFABGABESYSTEME
PROCÉDÉ DE PRODUCTION DE CRYOGELS AVEC DES BIOMATÉRIAUX NATURELS ET UTILISATION DE TELS CRYOGELS EN TANT QU' ÉCHAFAUDAGES DE TISSU MOU OU SYSTÈMES D'ADMINISTRATION DE MÉDICAMENT

(30) Priority: 17.09.2019 TR 201914116
(43) Date of publication of application: 27.07.2022
(73) Proprietor: Yeditepe Universitesi, 34755 Istanbul (TR)
(72) Inventor: BEKTAS TAS, Ezgi Irem, 34755 Atasehir/Istanbul (TR); GÜREL PEKÖZER, Görke, Istanbul (TR); TORUN KÖSE, Gamze, 34755 Istanbul (TR); KÖK, Fatma Nese, 34469 Istanbul (TR); AYTEKIN, Ali Özhan, 34755 Istanbul (TR)
(74) Representative: Yalçiner Patent and Consulting Ltd.
(86) International application number: PCT/TR2020/050848
(87) International publication number: WO 2021/054921

(56) References cited:
- EP-A1- 3 533 861
- WO-A1-03/015745
- AU-A1- 2013 204 780
- CA-A1- 2 198 569
- US-B1- 6 309 661

## Description

### Field of the Invention

The present invention relates to use of cryogels obtained from natural biomaterials for the production of drug delivery systems, or for the production of cell-based or cell-free soft tissue scaffolds.

### Background of the Invention

Tissue engineering is an emerging field that has the potential to provide permanent solutions to millions of people who have suffered tissue damage or loss. Basic components of tissue engineering are cells, natural or artificial polymeric scaffolds and stimulating factors. The materials can be used alone or in combination with other tissue engineering elements. Orthopedic solutions in use today are invasive surgical methods, tissue or cell transfers and various implants. However, these methods may have disadvantages such as long recovery periods, immunological rejection, and toxicity.

Artificial polymeric scaffolds show lower biocompatibility compared to natural ones. In addition, acidic degradation products of artificial polymeric scaffolds cause toxicity. **Therefore, natural biomaterials are used in our invention.** In addition to these, in most of the methods used during production of tissue scaffolds, problems are encountered such as failure to provide sufficient pore size and porosity, high degradation rate of the materials, low mechanical properties, and the fact that the formed scaffold structures are not formed in the same way when repeated.

Locust bean gum (LBG) obtained from locust bean fruit, xanthan gum (XG) produced by *Xanthomonas campestris* bacteria and mastic gum (MG) produced by gum tree called *Pistacia lentiscus* are generally used in applications in the fields of food and pharmacy.

When the literature was reviewed, **it was seen that the hydrogels or cryogels prepared in LBG-XG structure were not used in any tissue engineering application.** In the published articles, it has been observed that the LBG-XG mixture was only used in the formation of structures such as edible films, tablets or microparticles for drug delivery purposes **[1, 2, 3].**

Upon searching the related patent studies, the patent document numbered CA1093466 (A) discloses various LBG-XG compositions developed for drug delivery system, particularly for ophthalmic drugs. The patent document numbered EP0818991 (A1) mentions that LBG and XG are used in the controlled release powder insufflation formulations. The patent document numbered PT78813 (A) discloses about preparation of pharmaceutical compositions comprising LBG and XG. CA 2 198 569 (A1) describes a sponge which can be used as a wound dressing comprising a mixture of xanthan gum and locust bean gum. The weight ratio of these compounds ranges from 1:99 to 99:1. The sponge may further comprise a therapeutic agent. The mixture of the compounds is frozen at -30°C for at least 20 minutes, followed by freeze-drying to form the sponge.

When the papers and patents in the literature were reviewed, it was seen that there was no drug delivery system or a scaffold structure that could be used in the field of tissue engineering consisting of LBG-XG-MG mixture.

### Summary of the Invention

The objective of the present invention is to produce a porous tissue scaffold from natural and biocompatible materials such as LBG, XG and MG, and to provide an environment for cells to adhere to and grow on these scaffolds, and if needed, for release of molecules such as drugs, growth factors, hormones, etc.

Another objective of the invention is to use materials prepared with LBG, XG and MG, which are natural and biocompatible materials, as cell-free tissue support.

A further objective of the invention is to use the biomaterials that will be prepared with LBG, XG and MG for production of a drug delivery system that provides extended release of the molecule loaded therein.

### Detailed Description of the Invention

**"Method of Producing Cryogels with Natural Biomaterials and Use of Such Cryogels as Soft Tissue Scaffolds or Drug Delivery Systems"** developed to fulfill the objective of the present invention is illustrated in the accompanying figures, in which;
- **Figure 1.**: is a representation of the graph of a 60-day percent weight loss of 2% (weight/volume) LBG, XG, LBG-XG, LBG-XG-MG cryogels.
- **Figure 2.**: is a representation of the graph of a 60-day percent weight loss of 3% (weight/volume) LBG, XG, LBG-XG, LBG-XG-MG cryogels.
- **Figure 3.**: is a representation of the graph of a 60-day percent weight loss of only LBG and XG comprising cryogels.
- **Figure 4.**: is a representation of the graph of a 60-day pH change of 2% (weight/volume) LBG, XG and MG containing cryogels in phosphate buffer saline solution.
- **Figure 5.**: is a representation of the graph of a 60-day pH change of 3% (weight/volume) LBG, XG and MG containing cryogels in phosphate buffer saline solution.
- **Figure 6.**: is a representation of the graph of a 60-day pH change of cryogels containing only LBG or XG.
- **Figure 7.**: is a representation of the SEM micrographs of the samples containing 2% LBG-XG prepared at a ratio of 1:1 and 2:1 by mass and samples obtained by adding 5 mg/mL and 10 mg/mL MG to the said samples.
- **Figure 8.**: is a representation of the SEM micrographs of the samples containing 3% LBG-XG prepared at a ratio of 1:1 and 2:1 by mass and samples obtained by adding 5 mg/mL and 10 mg/mL MG to the said samples.
- **Figure 9.**: is a representation of the graph of pore size distribution of the samples 1-12 given in Table 1.
- **Figure 10.**: is a graphical representation of the FTIR results received for LBG, XG and MG.
- **Figure 11.**: is the FTIR spectrum of samples containing 2% and 1:1 LBG-XG (MG ratio 0 mg/mL,5 mg/mL ve 10 mg/mL respectively).
- **Figure 12.**: is the FTIR spectrum of samples containing 3% and 1:1 LBG-XG (MG ratio 0 mg/mL,5 mg/mL ve 10 mg/mL , respectively).
- **Figure 13**.: is the FTIR spectrum of samples containing 2% and 2:1 LBG-XG (MG ratio 0 mg/mL,5 mg/mL ve 10 mg/mL respectively).
- **Figure 14**.: is the FTIR spectrum of samples containing 3% and 2:1 LBG-XG (MG ratio 0 mg/mL,5 mg/mL ve 10 mg/mL respectively).
- **Figure 15**.: is a graphical representation of the result of the tensile analyses of cryogels (Young's Modulus).
- **Figure 16.**: is a graphical representation of the result of the shear analyses of cryogels (Young's Modulus).
- **Figure 17.**: is a graphical representation of the time-dependent change in the cumulative concentration of Kartogenin released from cryogels.
- **Figure 18.**: is a graphical representation of the time-dependent change in the percentage of cumulative release of Kartogenin released from cryogels.
- **Figure 19.**: is a graphical representation of the viability test of the cells seeded on the tissue scaffolds.

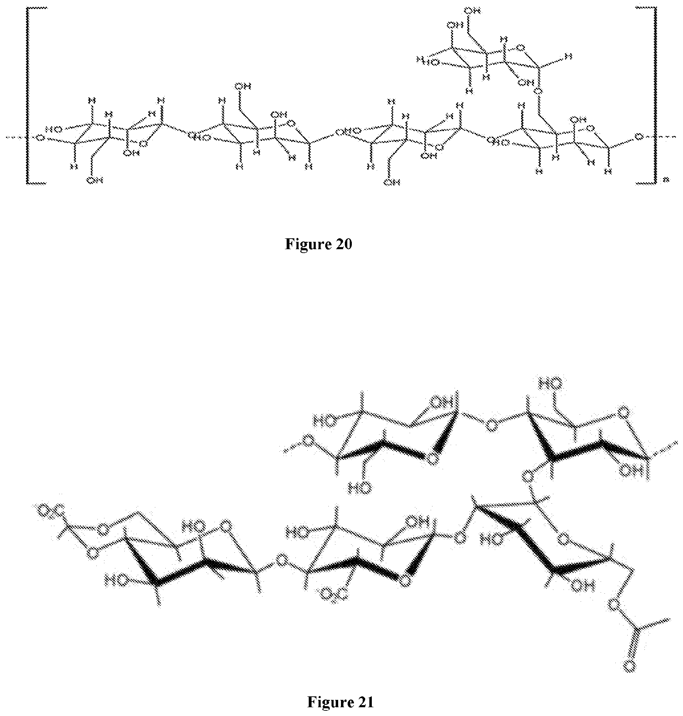

- **Figure 20.**: is the representation of the chemical structure of LBG (Locust bean gum).
- **Figure 21.**: is the representation of the chemical structure of XG (Xanthan gum).

Within the scope of the present invention, it is aimed to produce a porous tissue scaffold from natural and biocompatible materials such as LBG, XG and MG, and this scaffold is aimed to provide an environment for cells to adhere thereto and grow thereon. In addition, the tissue scaffold system is aimed to also act as a drug delivery system that provides extended release of the molecule loaded therein. The invention developed accordingly is related to the fields of material engineering, biomaterials, tissue engineering, bioengineering and biotechnology.

The invention is completely produced from natural materials. When these materials are combined, macroporous hydrogel (cryogel) scaffolds are formed. Hydrogels are three-dimensional and are tissue scaffolds with high water holding capacity. They can be used in the form of films, microparticles, nanoparticles, coatings or tissue scaffolds. The porous structure of the product of the invention is suitable for loading micro- or macro-molecular drug structures. It can also provide long-term drug release depending on the diffusion rate.

LBG is produced by grinding locust bean seeds to obtain a fine particle size flour, followed by precipitation and purification thereof. It is a natural polymer that is biocompatible, biodegradable, excretable and edible. It is classified by the US Food and Drug Administration (FDA) under the GRAS category, which means generally recognized as safe. LBG can be degraded by the enzymes β-mannanase, β-mannisodase and α-galactosidase and the degradation products can be easily excreted from the body. Turkey is # 6 in the production of locust bean fruit in the world with a share of 6%. XG, on the other hand, is a polyanionic exopolysaccharide produced by the gram-negative pathogenic bacterium *Xanthomonas campestris,* and it is a stable material within a wide temperature range and is resistant to shear force. It is a biocompatible, reproducible, non-toxic substance with high water solubility. It has been approved by the US Food and Drug Administration as a food supplement without any quantity limits. In addition, it is used in many areas from the petroleum industry to the pharmaceutical industry and the production of cosmetic products. Mastic gum (MG) that is produced by the mastic tree called Pistacia lentiscus, which grows in the western Aegean (between 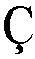 e 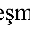 me and Karaburun) in our country, is a material that is known for its healing properties, especially used in gastrointestinal disorders. As a result of the recent studies, it is known that mastic resin is antimicrobial, antifungal, antioxidant, anti carcinogenic and anti-inflammatory. Taking all these features into consideration, macroporous hydrogels consisting of LBG, XG and MG are very suitable for use in soft tissue engineering and as drug delivery systems due to their biodegradability, biocompatibility, inexpensiveness, renewability and reproducibility.

A method developed within the scope of the invention for preparation of cryogels from LBG-XG natural biomaterials, wherein the following process steps are carried out:
- Weighing LBG and XG biomaterials at a ratio of 1:1 - 3:1 (preferably 1:1, 2:1 and 3:1) (weight/weight) relative to each other, and, by gradually mixing in distilled water, dissolving to obtain a solution in the concentration range of 1-4% ( weight/volume),
- Stirring the solution continuously for 30 minutes with the help of a magnetic stirrer while keeping the temperature in the range of 90-100°C,
- After the solutions are transferred to glass petri dishes, keeping them in the refrigerator at -20°C for 24 hours,
- Drying completely by applying lyophilization process at the final stage,
- Following the drying process via lyophilization, obtaining the cryogels as the final products.

A method developed within the scope of the invention for preparation of cryogels from LBG-XG-MG natural biomaterials, wherein the following process steps are carried out:
- Firstly, mixing MG with a magnetic stirrer in the concentration range of 0.1-50 mg/mL,
- Following this stage, weighing LBG and XG biomaterials at a ratio of 1:1 - 3:1 (preferably 1:1, 2:1 and 3:1) (weight/weight) relative to each other, and, adding them into the MG solution to obtain a solution in the concentration range of 1-4% (weight/volume) (preferably 2% and 3% (weight/volume)),
- Gradually dissolving the solution in distilled water while keeping the temperature within the range of 90-100°C,
- After stirring the solutions continuously for 30 minutes, pouring them into glass petri dishes,
- Rapidly placing the mixture in the refrigerator at -20°C and storing for 24 hours,
- Applying lyophilization process on the tissue scaffolds in order to remove the solvent,
- After drying with lyophilization, obtaining cryogels, which are the final products.

These cryogels obtained within the scope of the present invention can be used for the production of cell-based or cell-free scaffolds. In addition, these cryogels can be used for the production of a drug delivery system for drug delivery purposes upon encapsulating substances such as drug molecules, small molecules, growth factors, etc.

### Experimental Studies

The experimental studies conducted within the scope of developing the present invention can be listed as follows:
- Topographic, mechanical and chemical characterizations of the cryogels produced from LBG-XG and LBG-XG-MG were carried out.
- Degradation analyses of the cryogels produced from LBG-XG and LBG-XG-MG were carried out.
- Small molecule release study of the cryogels produced from LBG-XG and LBG-XG-MG was conducted.
- Viability study was conducted by seeding cells on the cryogels produced from LBG-XG and LBG-XG-MG.

### 1. Preparing Cryogels from LBG-XG

It was observed that when cryogels were prepared separately from LBG and XG biomaterials, weaker tissue scaffolds were obtained; therefore LBG and XG were gradually dissolved at ratios of 1: 1, 2: 1 and 3: 1 weight/weight in distilled water by stirring in a concentration range of 1-4% weight/volume. The solution was stirred continuously for 30 minutes with the help of a magnetic stirrer while keeping the temperature in the range of 90-100°C. After the solutions were transferred to glass petri dishes, they were placed in the refrigerator at -20°C and stored for 24 hours, and at the final stage, they were completely dried by applying lyophilization. The dried tissue scaffolds were cut to fit into 24-well plates and prepared for the analyses to be conducted.

In the first stage, LBG and XG were combined at a ratio of 1:1-2:1-3:1 (weight/weight) in a concentration range of 1-4% (weight/volume). Polymers with two and three percent concentrations were found to be more suitable for tissue scaffolds than polymers with other concentrations in terms of their density. Solutions with a concentration of four percent could not be homogeneously distributed to the glass petri dishes due to their high density, therefore they were found unsuitable for use as a tissue scaffold. As a result of these observations, the experiments were continued by concentrating on the samples with a total polymer concentration of two percent and three percent. Since it was observed in the previous cryogel trials that LBG polymer was more durable compared to XG, the experiments were continued by increasing the ratio of LBG by mass. At this stage, tissue scaffold trials were carried out by combining LBG-XG composites at a ratio of 2:1 and 3:1 by mass.

### 2. Preparing Cryogels from LBG-XG-MG

Cryogel trials were performed by combining LBG, XG and MG. Initially, MG was dissolved in distilled water by being stirred with a magnetic stirrer in the concentration range of 0.1-50 mg/mL and keeping the temperature in the range of 90-100°C. Following this step, LBG and XG were gradually dissolved at a ratio of 1:1 - 2:1 (weight/weight) by mass, in a concentration of 2% and 3% (weight/volume). After the solutions were stirred continuously for 30 minutes, they were poured into glass petri dishes and rapidly placed in the refrigerator at - 20°C and kept for 24 hours. Lyophilization process was applied on the tissue scaffolds in order to remove the solvent. LBG and XG were prepared in the same way at a ratio of 2:1 by mass. 5, 10, 25 and 50 mg/mL MG were added to the resulting solutions having concentrations of 2% and 3% (weight/volume) containing 1:1 and 2:1 ratios of LBG and XG by mass. It was observed that composites containing two percent LBG-XG (1:1) (weight/weight) and 25 mg/mL and 50 mg/mL MG were very dense. For that reason, studies were continued with polymers containing 5 mg/mL and 10 mg/mL MG. Tissue scaffolds have been successfully produced from 2% and 3% (weight/volume) polymers containing LBG-XG at a ratio of 2:1 by mass and 5 mg/mL or 10 mg/mL of MG.

### 3. Degradation Analysis of Cryogels

In the first stage, degradation analysis was performed for 16 samples. These samples are polymers with a total polymer concentration of 2% and 3% (weight/volume), containing 1:1 and 2:1 LBG-XG by mass and containing 5 mg/mL and 10 mg/mL MG, and cryogels containing only 2% and 3% (weight/volume) LBG or XG alone (Table 1). The cryogels produced for the degradation analysis were weighed and put into isotonic salt solution containing 0.09% sodium azide and kept in a shaking water bath at 37°C. The samples were taken out of the water bath on day 7, 15, and 30; the pH values of the solutions, in which they were present, were measured; and were lyophilized by freezing at - 80°C. Percent degradation graphs were generated upon finding the percent weight loss between the initial weights and final weights of the samples after they were completely dried.

**Table 1. Samples selected for the degradation analyses**

| **Sample Number** | **Sample content** |
|---|---|
| 1 | 2% (weight/volume - 1:1 (weight/weight) LBG-XG |
| 2 | 2% (weight/volume ) - 1:1 (weight/weight) LBG-XG + 5 mg/mL MG |
| 3 | 2% (weight/volume) - 1:1 (weight/weight) LBG-XG + 10 mg/mL MG |
| 4 | 2% (weight/volume) - 2:1 (weight/weight) LBG-XG |
| 5 | 2% (weight/volume) - 2:1 (weight/weight) LBG-XG + 5 mg/mL MG |
| 6 | 2% (weight/volume) - 2:1 (weight/weight) LBG-XG + 10 mg/mL MG |
| 7 | 3% (weight/volume) - 1:1 (weight/weight) LBG-XG |
| 8 | 3% (weight/volume) - 1:1 (weight/weight) LBG-XG + 5 mg/mL MG |
| 9 | 3% (weight/volume) - 1:1 (weight/weight) LBG-XG + 10 mg/mL MG |
| 10 | 3% (weight/volume) - 2:1 (weight/weight) LBG-XG |
| 11 | 3% (weight/volume) - 2:1 (weight/weight) LBG-XG + 5 mg/mL MG |
| 12 | 3% (weight/volume) - 2:1 (weight/weight) LBG-XG + 10 mg/mL MG |
| 13 | 2% (weight/volume) LBG |
| 14 | 3% (weight/volume) LBG |
| 15 | 2% (weight/volume) XG |
| 16 | 3% (weight/volume) XG |

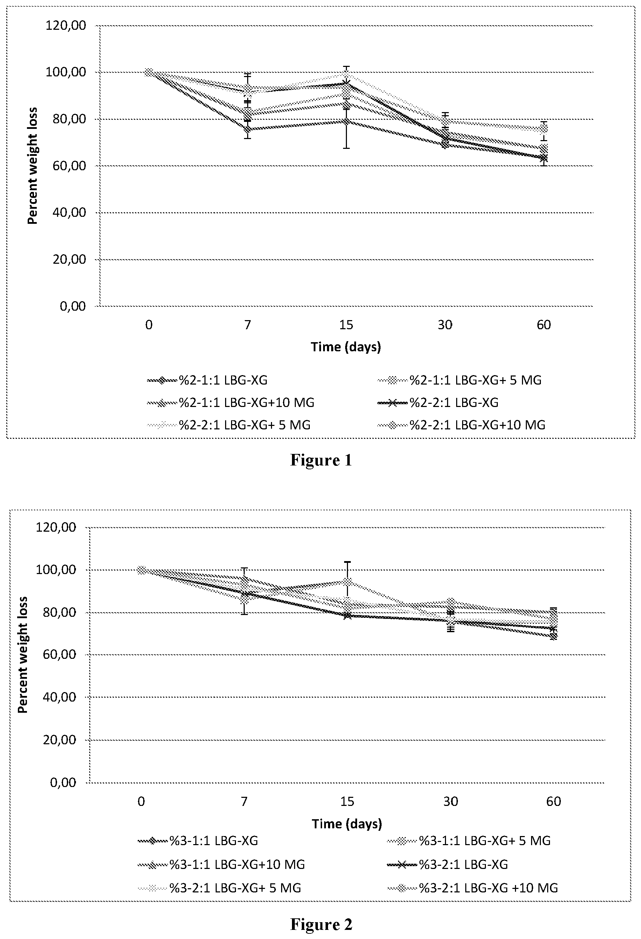

When the percent weight loss graphs of cryogels were examined, the weight loss of the samples containing 2% (weight/volume) LBG-XG at the end of day 60 was in the range of 24% - 37%, while the weight loss of the samples containing 3% (weight/volume) LBG-XG was in the range of 23% - 32% (Figure 1 and 2). The highest percent weight loss was observed in 2% (weight/volume) - 1:1 (weight/weight) and 2% (weight/volume) - 2:1 (weight/weight) LBG-XG samples (37%), while the lowest weight loss was observed in 3% (weight/volume) - 1:1 (weight/weight) LBG-XG + 10 mg/mL MG sample (20%). When Figure 1 and Figure 2 were compared, it was determined that the samples containing 2% (weight/volume) LBG-XG lost more weight at the end of day 60 than the samples containing 3% (weight/volume) LBG-XG. It was observed that percent weight loss in cryogels containing the same concentration of LBG-XG was lower in the samples containing MG than those which did not. No major difference was observed in percent weight loss values between samples containing different concentrations of MG.

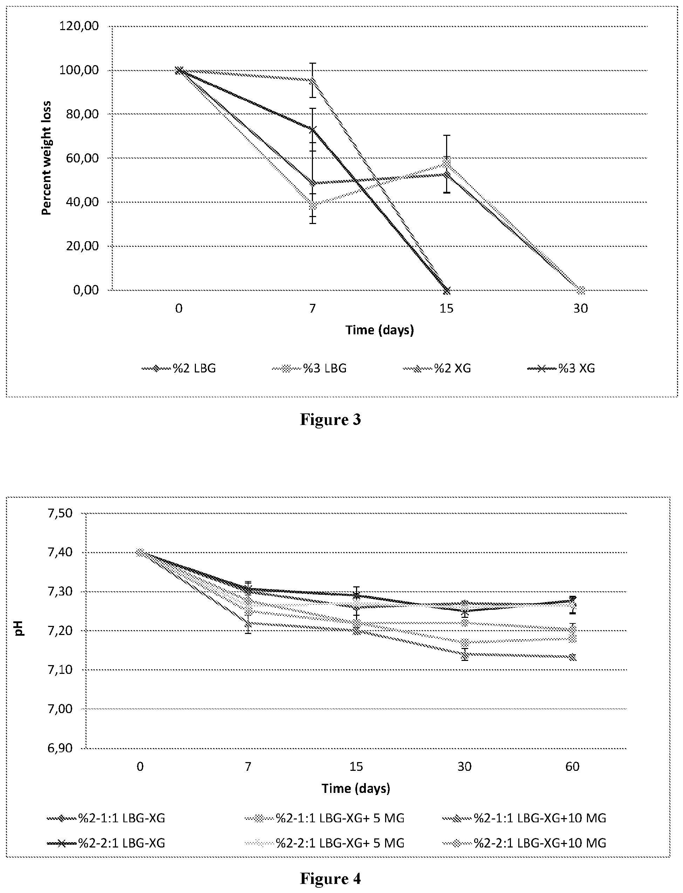

When the degradation analysis of cryogels containing only XG or only LBG was examined, it was observed that these cryogels completely degraded on day 15 and 30, respectively (Figure 3). According to this result, the degradation rate of XG is higher than LBG.

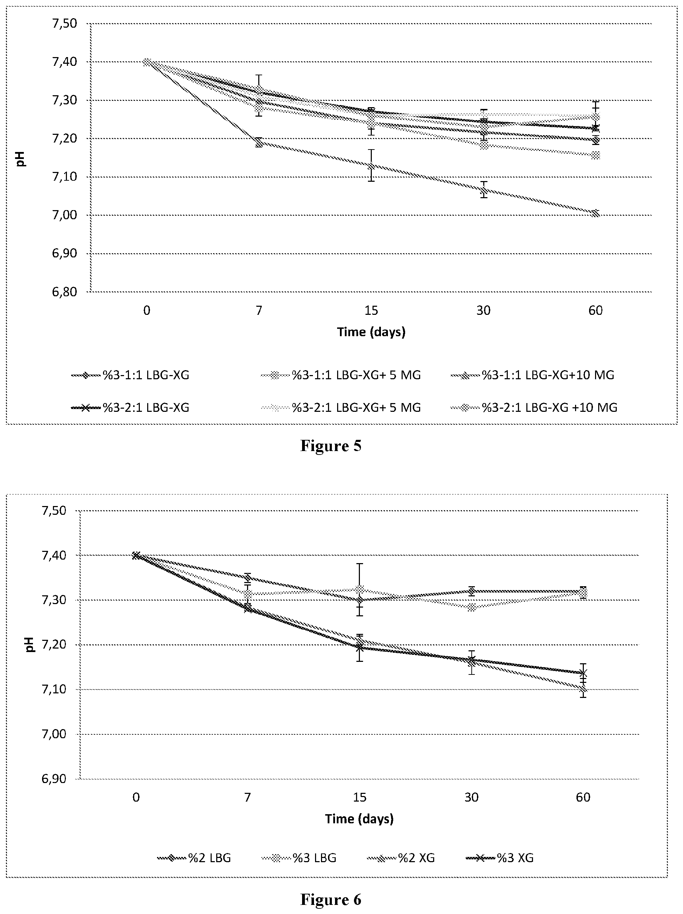

During the degradation analysis, while determining the percent weight loss, the pH values of the solutions, in which the cryogels were kept, were also determined. A significant decrease in the pH value of the region where the tissue scaffold is implanted is undesirable because it causes necrosis in the cells and surrounding tissues. For this reason, the changes in the pH of the medium caused by the products released during the degradation of cryogels should be examined. When the first 12 samples in Table 1 were compared, the sample with the lowest pH at the end of 60 days was the 3% (weight/volume) - 1:1 (weight/weight) LBG-XG + 10 mg/mL MG (pH: 7.06)), and the sample with the highest pH was 2% (weight/volume) - 2:1 (weight/weight) LBG-XG (pH: 7.27) (Figures 4 and 5). When the percentage weight loss and pH analysis were compared, it was determined that the pH of the samples with the highest percent weight loss among 12 samples was closest to the initial pH value of 7.4. It was determined that 3% (weight/volume) - 1:1 (weight/weight) LBG-XG + 10 mg / mL MG, which is one of the samples with the least loss in the percent weight loss ranking, has the lowest pH (7.06). The reason for this is thought to be the acidic chemicals in the structure of MG, and glucuronic acid and pyruvic acid in the structure of XG which are released upon degradation. In general, it was determined that the pH of the samples containing MG was lower than the other samples, but at the end of day 60, none of the pH values determined fell below 7. For this reason, this decrease in the pH values was not considered as a significant decrease.

When the pH change of the cryogels containing only LBG or XG at the end of day 60 was examined (Figure 6), it was seen that the pH of the cryogels consisting of XG was lower than the pH of the cryogels consisting of LBG. This is caused by glucuronic acid and pyruvic acid, which are in the structure of XG and released as a result of degradation.

### 4. Scanning Electron Microscopy (SEM) Analysis

Scanning electron microscopy analysis was performed in order to observe the surface structure of the cryogels. Completely dried samples were coated with 10 nm thick gold for analysis, and then imaged under high vacuum and 10 kV high voltage with Carl Zeiss EVO-40 device at magnification of 100-500 times. SEM analysis allowed observing the surface characteristics of LBG, XG and LBG-XG-MG samples.

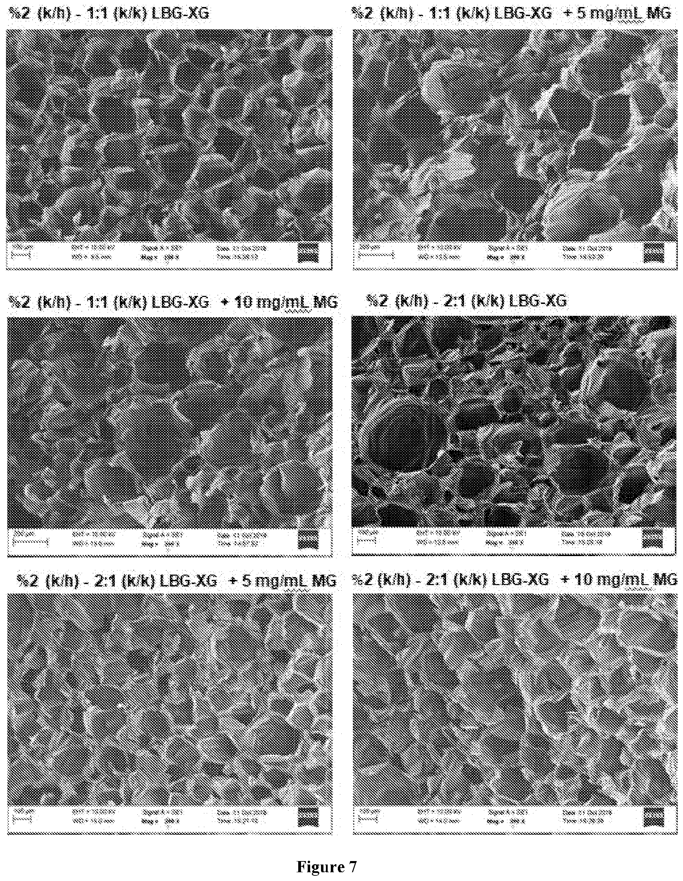

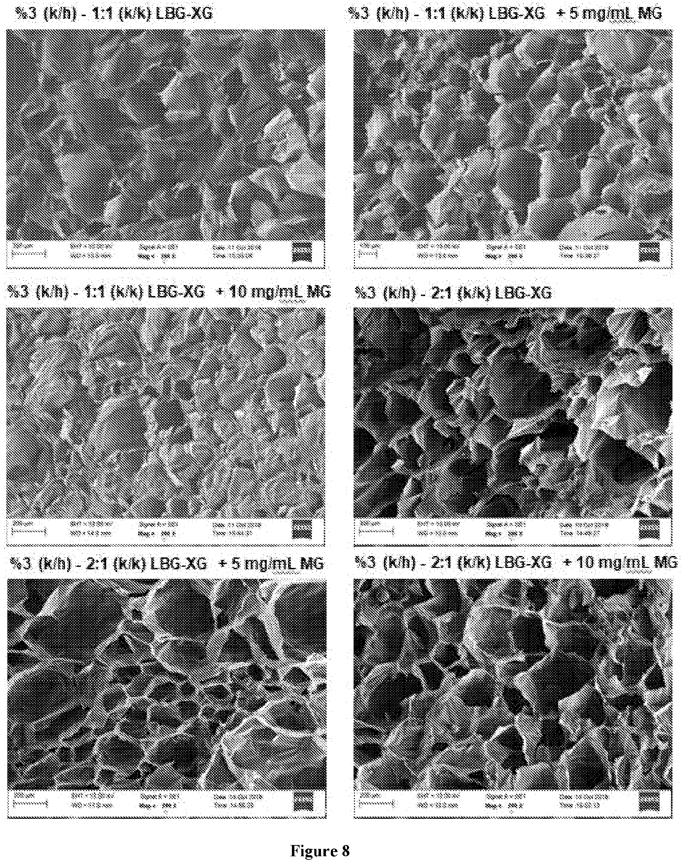

SEM micrographs show the interconnected porous structures of cryogels composed of LBG-XG and LBG-XG-MG. 5 mg/mL and 10 mg/mL MG were added to cryogels containing 2% and 3% LBG-XG by mass, and SEM analysis thereof was performed. Topographic features and porous structures of the samples were observed in the micrographs. It was determined that the amount of MG did not affect the porous structure (Figures 7 and 8).

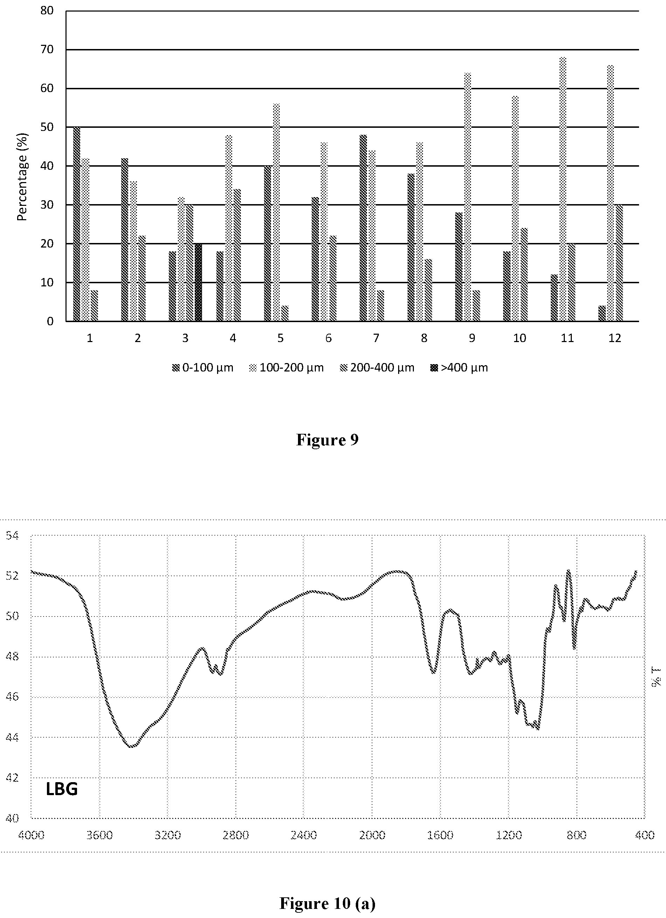

As a result of the degradation analysis, since the samples containing only LBG and only XG degrade faster than the other samples, it was decided to limit the number of samples to the first twelve samples in Table 1, and to continue the characterization studies with these samples from this section onwards. The pore sizes of the materials were analyzed using SEM micrographs and their pore size distributions were determined by the pore analysis program written in C-Sharp programming language. In the program, the size of 50 randomly selected pores was measured for each sample. The measurements were categorized as the number of pores corresponding to 0-100 µm, 100-200 µm, 200-400 µm and ≥400 µm ranges. The results are given in Figure 9 as a percent distribution.

According to Figure 9, the pore sizes of the cryogels are below 400 µm except for the 3^{rd} sample. Another observation is that samples with pores in the range of 100-400 µm (82-96%), most of which are in the range of 100-200 µm (58-66%), are samples containing 3% 2:1 LBG: XG (Sample 10-12). The pore sizes of these samples are more homogeneous compared to the others. At the other end, there is a set of samples containing 2% 1:1 LBG:XG (Sample 1-3). In the range of 100-400 µm, the pore ratio decreases to 50-62%. As a result of the SEM analysis, it was observed that the pore sizes of all samples with a ratio of 2:1 were higher when compared to the samples with a ratio of 1:1. It has been found that the increase in the LBG concentration increases the pore size. As a result of this analysis, it was seen that the cryogels produced generally have pore sizes suitable for soft tissue formation.

### 5. FT-IR Analysis

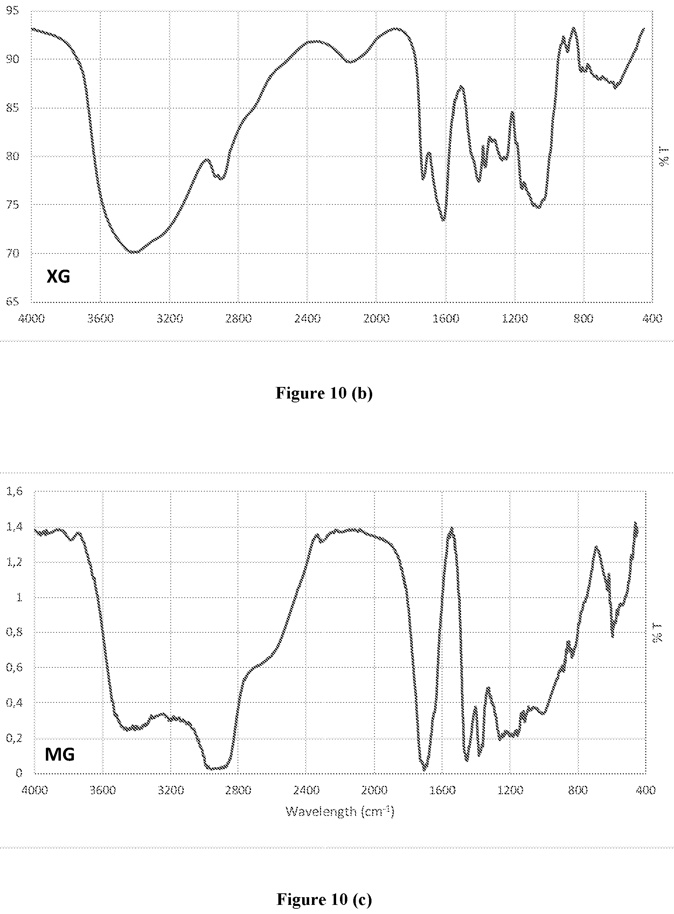

FTIR analyses were performed for the selected samples (The first 12 samples given in Table 1). In Figure 10, FTIR results of the three materials used are shown separately.

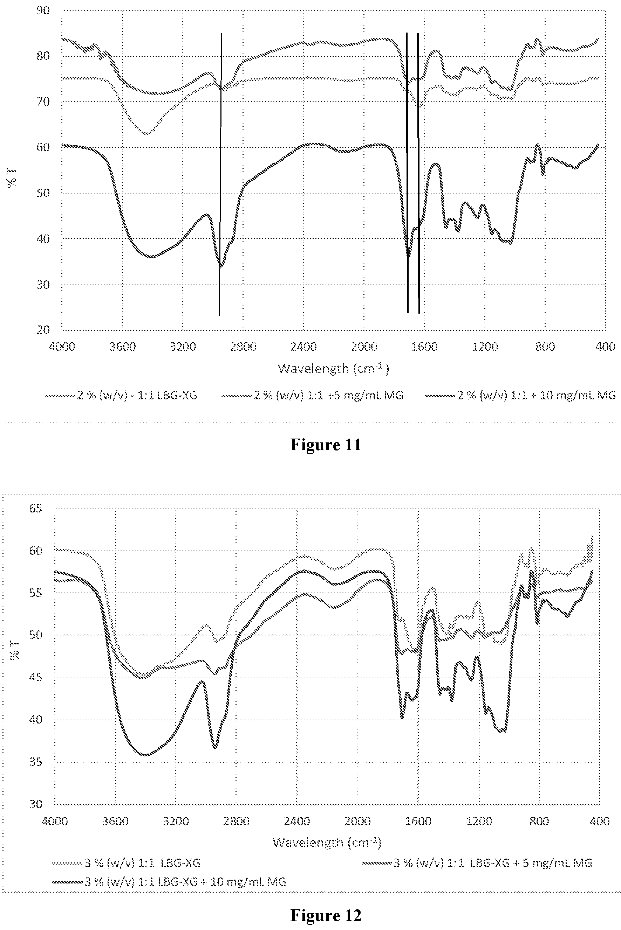

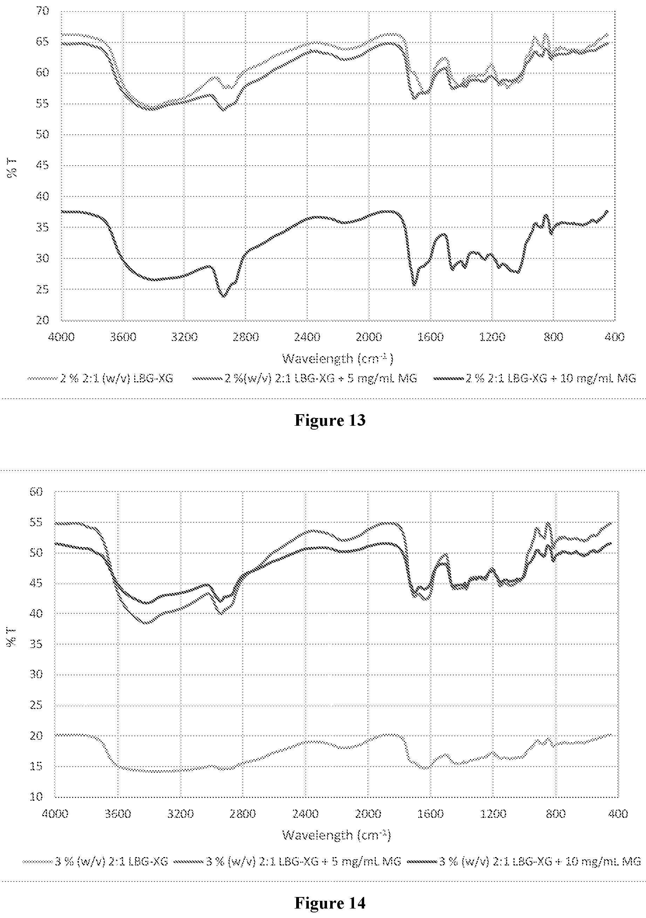

In the following 4 figures (Figures 11-14), the results of the 4 sample sets formed are given respectively. In each graph, % polymer content and LBG-XG ratio are the same, and the 3 different curves show samples with increasing amounts of MG (0%, 5% and 10%).

Some bands that significantly differ upon MG addition are marked by vertical lines in Figure 11. These bands show similar tendency to change in the other 3 sample sets as well. Although CH₂ asymmetric tension in the 2900-2940 cm⁻¹ band and CH₃ symmetric tension around 2870 cm⁻¹ band are present in all samples, it becomes more apparent in each sample set with the addition of MG, and the 2900-2940 cm⁻¹ band becomes significantly more dominant. The other marked regions 1731 cm-1 and 1614 cm⁻¹ indicate the carbonyl (C = O) tension, and it appears that the peak of 1731 cm⁻¹ increased proportionally to the MG addition in all four sample groups. On the other hand, the peak of 1614 cm⁻¹, which seems to be more dominant in pure LBG and XG samples, decreases relatively with the addition of MG. Between 1200 cm⁻¹ - 970 cm⁻¹, there is C-C and C-O tension of the pyranose ring and C-O-C tension of glycosidic bonds. These bands are included in all samples.

### 6. Mechanical Tests

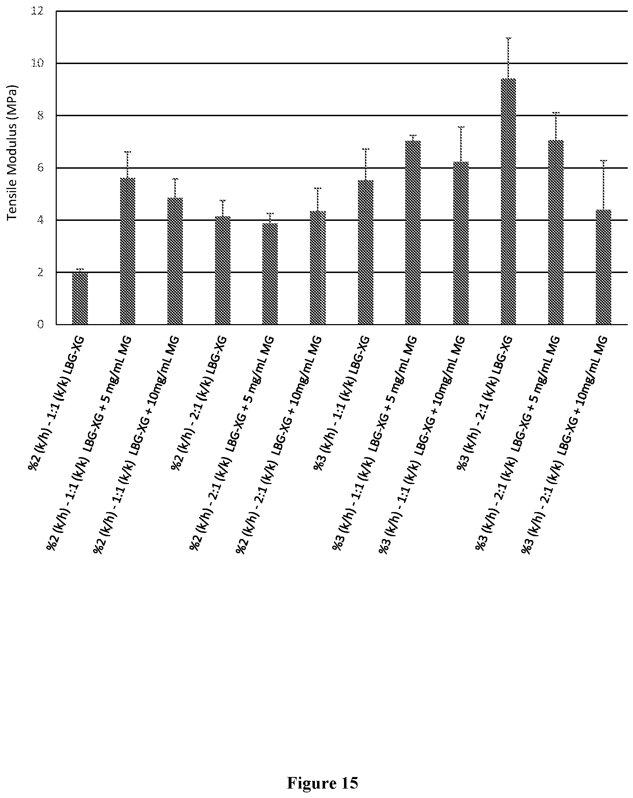

The tensile test analysis results of the cryogels are present in Figure 15. As can be seen, increase in the MG ratio decreases the tensile modulus values. While the Young's Modulus values were observed to decrease in LBG-XG samples with total polymer concentration of 2% (weight/volume) 1-1 and 3% (weight/volume) 1-1 as the amount of MG increased, it was observed that MG had no significant effect on Young's Modulus values in 2% (weight/volume) LBG-XG samples mixed at a ratio of 2:1. The highest Young's Modulus value was observed in 3% (weight/volume) 2:1 sample. All of the cryogels have Young's Modulus (for tensile test) of 2-10 MPa.

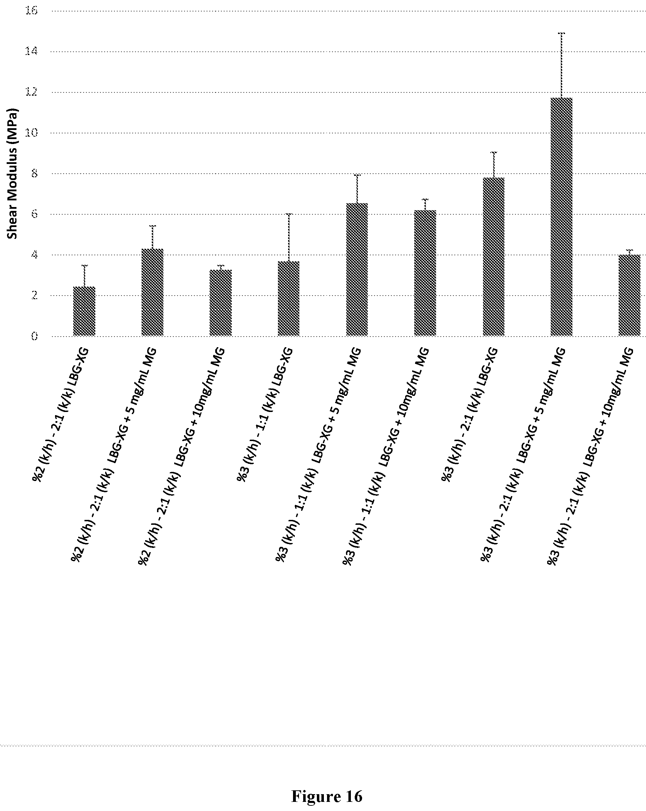

The mechanical device pulling speed was set at 0.08 N/min for the shear modulus analysis of cryogels. The shear modulus is a value used to determine the rigidity of materials, indicating the resistance of the materials against the uniaxial transverse force applied parallel to the surface of the materials. When the shear modulus values of the cryogels were compared, it was observed that the highest value was in the cryogels consisting of 3% (weight/volume) - 2:1 (weight/weight) LBG-XG + 5 mg/mL MG (Figure 16). Looking at the shear modulus values of all cryogels, it was seen that the value increased when 5 mg of MG was added to 2% - 1:1 and 3% - 1:1 samples, but when more MG was added, the shear modulus value decreased. When 2% - 2:1 and 3% - 2:1 samples were evaluated, it was found that increase in the amount of MG decreased the shear modulus values. When the cryogel samples containing 2% and 3% polymer by mass were compared, it was determined that the shear modulus values of the samples with 3% were generally higher than the samples with 2%.

### 7. Encapsulation small molecule (Kartogenin) in cryogel and characterization of the release

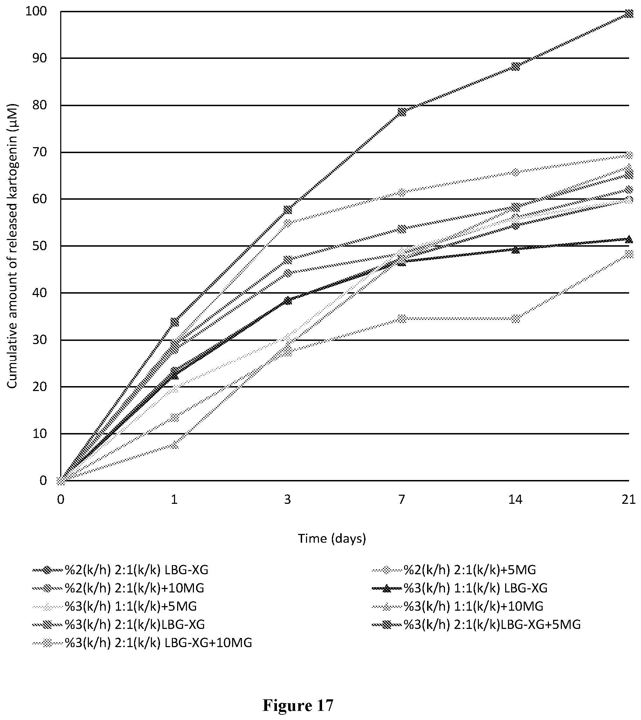

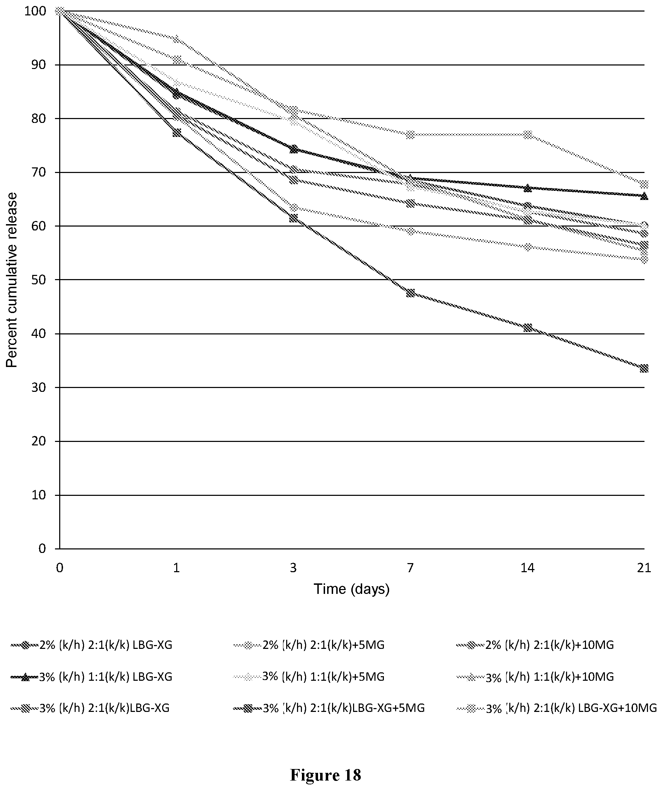

Cryogels consisting of LBG-XG and LBG-XG-MG were combined at concentrations of 2% - 3% (weight/volume) and at ratios of 1:1 - 2:1 (weight/weight) and dissolved in distilled water with the help of a magnetic stirrer for 30 minutes keeping the temperature at 90-100°C temperature. After the solutions were transferred to glass petri dishes, they were kept in the refrigerator at -20°C for 24 hours. In the last stage, the samples were dried by means of a lyophilizer. The small molecule Kartogenin, which induces cartilage formation, was added such that there will be 150 µM in 1 mL release medium (PBS) to the formed cryogels when in dry state, and was left overnight to be encapsulated in the cryogel. The next day, PBS-swollen cryogels were placed into 1 mL of release medium (PBS). On days 1, 3, 7, 14 and 21, absorbance was measured by taking samples from the release medium, and the amount of kartogenin that was released was calculated cumulatively and percent cumulatively (Figures 17 and 18) using the absorbance value and calibration curve. The PBS solution containing the cryogels was renewed after each measurement.

When Figures 17 and 18 were examined, it was observed that all samples were capable of prolonged release of kartogenin. A faster release was observed in the first 3 days in all samples compared to other time intervals. This is due to the fact that kartogenin absorbed on the cryogel surface easily passes into the release medium. After the third day, the release rate decreased as Kartogenin, encapsulated in the cryogel, started to be released. The low release rate until day 21 also indicates that the cryogels maintain their physical integrity. As a result of 21 days of release of cryogels, samples of 2% (weight/volume) 2:1 (weight/weight), 3% (weight/volume) 1:1 (weight/weight) and 3% (weight/volume) 2:1 (weight/weight) containing 5 mg/mL MG released more Kartogenin (6%, 6% and 20%, respectively) than those that did not contain MG. It was observed that the release amount of Kartogenin in the 2% (weight/volume) - 2:1 (weight/weight) LBG-XG sample containing 10 mg/mL of MG did not change compared to the one not containing MG; and that the release amount in the 3% (weight/volume) - 1:1 (weight/weight) LBG-XG sample was more (11%) than the one not containing MG and the release amount in the 3% (weight/volume) - 2:1 (weight/weight) LBG-XG sample was less (16%) than the one not containing MG. When the samples containing 5 mg/mL of MG and 10 mg/mL of MG were compared, it was determined that in the 2% (weight/volume) - 2:1 (weight/weight) LBG-XG and 3% (weight/volume) - 2:1 (weight/weight) LBG-XG samples, the samples containing 5 mg/mL MG released more kartogenin (5% and 36%, respectively), while in the 3% (weight/volume) - 1:1 (weight/weight) LBG-XG sample, the samples containing 10 mg/mL of MG released 5% more kartogenin.

At the end of 21 days of incubation, it was observed that all samples released approximately 23-46% of Kartogenin except the 3% (weight/volume) - 2:1 (weight/weight) 5 mg/mL MG sample. The 3% (weight/volume) - 2:1 (weight/weight) 5 mg/mL MG sample released 40% of its Kartogenin at the end of day 7 and about 60% at the end of day 21. According to these results, all samples were able to sustain the release of Kartogenin for 21 days. It was determined that cryogels are delivery systems that are able to release for a desired period of time. The cryogels that are formed can release small molecules, growth factors, and various drugs.

### 8. MTS Cell Viability Assay

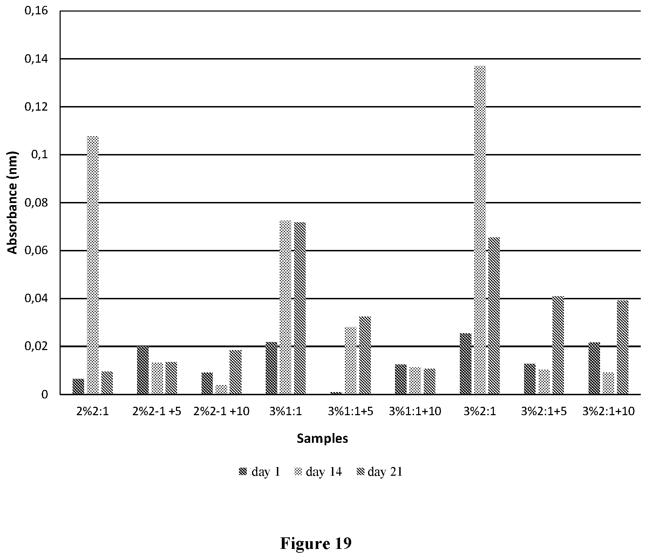

The interaction of cryogels with cells was examined by MTS assay. 60,000 cells were seeded on each cryogel, and cell viability test was performed on day 1, 14 and 21. According to the result of the assay, the cells adhered to all cryogels and were able to grow on them (Figure 19). It was determined that cryogels showed no toxic effects on the cells. Thus, it has been determined that cryogels have the potential to be used as tissue scaffolds in soft tissue formation.

### REFERENCES

[1].Kurt, Abdullah, Omer Said Toker, and Fatih Tornuk. 2017. "Effect of Xanthan and Locust Bean Gum Synergistic Interaction on Characteristics of Biodegradable Edible Film." International Journal of Biological Macromolecules 102: 1035-44. http://dx.doi.org/10.1016/j.ijbiomac.2017.04.081.
[2].Sharma, Navneet, Rohan D. Deshpande, Deepak Sharma, and Rakesh Kumar Sharma. 2016. "Development of Locust Bean Gum and Xanthan Gum Based Biodegradable Microparticles of Celecoxib Using a Central Composite Design and Its Evaluation." Industrial Crops and Products 82: 161-70. http://dx.doi.org/10.1016/j.indcrop.2015.11.046.
[3].Venkataraju, M., D. Gowda, K Rajesh, and H. Shivakumar. 2008. "Xanthan and Locust Bean Gum (from Ceratonia Siliqua) Matrix Tablets for Oral Controlled Delivery of Metoprolol Tartrate." Current Drug Therapy 3(1): 70-77.

## Claims

1. A method of producing soft tissue scaffolds with natural biomaterials in the form of cryogels from LBG (Locust bean gum) - XG (Xanthan gum) natural biomaterials, comprising the process steps of
∘ Weighing the LBG and XG at ratios of 1:1-3:1 relative to each other, and, by gradually mixing in distilled water, dissolving to obtain a solution in the concentration range of 1-4% (weight/volume),
∘ Stirring the solution continuously for 30 minutes with the help of a magnetic stirrer while keeping the temperature thereof in the range of 90-100°C,
∘ After the solutions are transferred to glass petri dishes, keeping them in the refrigerator at -20°C for 24 hours,
∘ Drying completely by applying lyophilization process at the final stage,
∘ Following the drying process carried out via lyophilization, obtaining the final cryogel tissue scaffolds.

2. A method of producing soft tissue scaffolds with natural biomaterials in the form of cryogels from LBG (Locust bean gum) - XG (Xanthan gum) - MG (Mastic Gum) natural biomaterials, comprising the process steps of
∘ Mixing MG in distilled water with a magnetic stirrer in the concentration range of 0.1-50 mg/mL,
∘ Following this step, adding LBG and XG at ratios of 1:1-3:1 (weight/weight) by mass relative to each other, and adding them into the MG solution to obtain a solution in the concentration range of 1-4% (weight/volume),
∘ Gradually dissolving the solution in distilled water while keeping the temperature thereof within the range of 90-100°C,
∘ After stirring the solutions continuously for 30 minutes, pouring them into glass petri dishes,
∘ Rapidly placing the mixture in the refrigerator at -20°C and storing for 24 hours,
∘ Applying lyophilization process on the tissue scaffolds in order to remove the solvent,
∘ Following the drying process carried out via lyophilization, obtaining the final cryogels tissue scaffolds.

3. A cryogel production method according to Claim 1 or Claim 2, **characterized in that** the ratio of LBG to XG is selected to be 1:1, 2:1 or 3:1.

4. Cryogels obtained by means of a method of Claim 1 or Claim 2.

5. Use of the cryogels of claim 4, for production of cell-based or cell-free soft tissue scaffolds.

6. Use of the cryogels of claim 4, for production of a drug delivery system by encapsulation of drug molecules, small molecules, or growth factors.

## Patentansprüche

1. Verfahren zur Herstellung von Weichgewebsgerüsten mit natürlichen Biomaterialien in Form von Kryogelen aus natürlichen Biomaterialien LBG (Johannisbrotkernmehl) - XG (Xanthan-Gummi), umfassend die Prozessschritte
∘ Wiegen von LBG und XG im Verhältnis 1:1-3:1 zueinander und Lösen durch allmähliches Einmischen von destilliertem Wasser unter Erhalt einer Lösung im Konzentrationsbereich 1-4%(Gewicht/Volumen),
∘ Rühren der Lösung kontinuierlich für 30 Minuten mit Hilfe eines Magnetrührers, wobei die Temperatur im Bereich von 90-100°C gehalten wird,
∘ Nachdem die Lösungen in Glas-Petrischalen überführt wurden, lagern Sie sie 24 Stunden lang bei -20°C im Kühlschrank,
∘ Vollständige Trocknung durch Anwendung des Gefriertrocknungsprozesses in der Endphase,
∘ Nach dem Trocknungsprozess, der durch Gefriertrocknung durchgeführt wurde, werden die endgültigen Kryogel-Gewebegerüste erhalten.

2. Verfahren zur Herstellung von Weichgewebsgerüsten mit natürlichen Biomaterialien in Form von Kryogelen aus natürlichen Biomaterialien LBG (Johannisbrotkernmehl) - XG (Xanthan-Gummi) -MG (Mastix-Gummi), umfassend die Prozessschritte
∘ Mischen von MG in destilliertem Wasser mit einem Magnetrührer im Konzentrationsbereich von 0.1-50 mg/mL,
∘ Nach diesem Schritt werden LBG und XG in einem Massenverhältnis von 1:1-3:1 (Gewicht/Gewicht) zueinander zugegeben und in die MG-Lösung gegeben, um eine Lösung im Konzentrationsbereich von 1-4% (Gewicht/Volumen) zu erhalten,
∘ Lösen Sie die Lösung allmählich in destilliertem Wasser auf, während Sie ihre Temperatur im Bereich von 90-100°C halten,
∘ Nachdem die Lösungen 30 Minuten lang kontinuierlich gerührt und in Glas-Petrischalen gegossen wurden,
∘ Schnelles Einlegen der Mischung in den Kühlschrank bei -20°C und Lagerung 24 Stunden,
∘ Anwendung eines Gefriertrocknungsprozesses auf den Gewebegerüsten, um das Lösungsmittel zu entfernen,
∘ Im Anschluss an den durch Gefriertrocknung durchgeführten Trocknungsprozess werden die endgültigen Kryogel-Gewebegerüste erhalten.

3. Kryogel-Herstellungsverfahren nach Anspruch 1 oder Anspruch 2, **dadurch gekennzeichnet, dass** das Verhältnis von LBG zu XG 1:1,2: 1 oder 3:1 gewählt wird.

4. Kryogele, die mittels eines Verfahrens Anspruch 1 oder Anspruch 2 erhalten werden.

5. Verwendung von Kryogelen nach Anspruch 4 zur Herstellung von zellbasierten oder zellfreien Weichgewebsgerüsten.

6. Verwendung von Kryogelen nach Anspruch 4 zur Herstellung eines Arzneimittelabgabesystems durch Verkapselung von Arzneimittelmolekülen, kleinen Molekülen oder Wachstumsfaktoren.

## Revendications

1. Procédé de production d'échafaudages de tissu mou avec des biomatériaux naturels sous forme de cryogels à partir de biomatériaux naturels LBG (gomme de caroube) - XG (gomme xanthane), comprenant les étapes de processus suivantes :
∘ Peser le LBG et le XG dans des proportions de 1:1 - 3:1 l'un par rapport à l'autre, et, en mélangeant progressivement à l'eau distillée, les dissoudre pour obtenir une solution dans une concentration comprise entre 1 - 4 % (poids/volume),
∘ Agiter la solution en continu pendant 30 minutes à l'aide d'un agitateur magnétique tout en maintenant sa température entre 90 - 100 °C,
∘ Une fois les solutions transférées dans des boîtes de Pétri en verre, les conserver au réfrigérateur à -20 °C pendant 24 heures,
∘ Sécher complètement en appliquant un processus de lyophilisation à la phase finale,
∘ Après le processus de séchage réalisé par lyophilisation, obtenir les échafaudages tissulaires cryogéniques finaux.

2. Procédé de production d'échafaudages de tissu mou avec des biomatériaux naturels sous forme de cryogels à partir des biomatériaux naturels LBG (gomme de caroube) - XG (gomme xanthane) - MG (gomme de mastic), comprenant les étapes de processus suivantes :
∘ Mélanger le MG à l'eau distillée avec un agitateur magnétique dans une concentration comprise entre 0.1 - 50 mg/mL,
∘ Après cette étape, ajouter de LBG et de XG dans des proportions de 1:1 - 3:1 (poids/poids) en masse l'un par rapport à l'autre, et les ajouter à la solution de MG afin d'obtenir une solution dans une concentration comprise entre 1 - 4 % (poids/volume),
∘ Dissoudre progressivement la solution à l'eau distillée tout en maintenant sa température entre 90 - 100 °C,
∘ Une fois les solutions agitées pendant 30 minutes en continu, les verser dans des boîtes de Pétri en verre,
∘ Placer rapidement le mélange au réfrigérateur à -20 °C et le conserver pendant 24 heures,
∘ Appliquer un processus de lyophilisation aux échafaudages tissulaires afin d'éliminer le solvant,
∘ Après le processus de séchage réalisé par lyophilisation, obtenir les échafaudages tissulaires cryogéniques finaux.

3. Procédé de production du cryogène selon la revendication 1 ou la revendication 2, **caractérisé en ce que** le rapport de LBG à XG est choisi pour être de 1:1, 2:1 ou 3:1.

4. Cryogels obtenus par le procédé selon la revendication 1 ou la revendication 2.

5. Utilisation des cryogels selon la revendication 4 pour la production d'échafaudages de tissu mou à base de cellules ou sans cellules.

6. Utilisation des cryogels selon la revendication 4 pour la production d'un système d'administration de médicaments par encapsulation de molécules de médicaments, de petites molécules ou de facteurs de croissance.
